Europäisches Patentamt

European Patent Office

Office européen des brevets

(11) Publication number: **0 196 669 B1**

# EUROPEAN PATENT SPECIFICATION

(45) Date of publication of patent specification: **12.02.92**

(51) Int. Cl.5: **C07K  17/06**, **A61K 49/02**, **A61K 49/00**, **A61K 43/00**

(21) Application number: **86104523.5**

(22) Date of filing: **02.04.86**

(54) **Improved method of conjugating metallothionein to biologically active molecules.**

(30) Priority: **03.04.85 US 719436**

(43) Date of publication of application:
**08.10.86 Bulletin  86/41**

(45) Publication of the grant of the patent:
**12.02.92 Bulletin  92/07**

(84) Designated Contracting States:
**AT BE CH DE FR GB IT LI LU NL SE**

(56) References cited:
**EP-A- 0 137 457**

(73) Proprietor: **THE DU PONT MERCK PHARMA-CEUTICAL COMPANY**
**Barley Mill Plaza, Building 25**
**Wilmington, Delaware 19880-0025(US)**

(72) Inventor: **Hadjian, Richard Albert**
**72 Beech Avenue**
**Melrose Massachusetts 02176(US)**
Inventor: **Drozynski, Cynthia Ann**
**404 Great Road**
**Stowe Massachusetts 01775(US)**

(74) Representative: **von Kreisler, Alek,**
**Dipl.-Chem. et al**
**Deichmannhaus am Hauptbahnhof**
**W-5000 Köln 1(DE)**

**Description**

FIELD OF THE INVENTION

This invention relates to an improved method for preparing conjugates of tracemetal-containing metallothionein and biologically active molecules.

BACKGROUND

Use of radiolabeled target-seeking biologically active molecules (hereinafter referred to as BAMs), especially antibodies and other proteins, for diagnostic and therapeutic purposes is a highly active field. For a discussion of such radiolabeling, see Eckelman et al., Radiolabeling of Antibodies, Cancer Research, 40:3036-3042 (1980) and Sfakianakis et al., Radioimmunodiagnosis and Radioimmunotherapy, J. Nucl. Med. 23:840-850 (1982). The most widely used means of radiolabeling antibodies has been direct iodination with I-131, I-125 or I-123. However, these radionuclides have certain dosimetric and imaging disadvantages. Certain metallic radionuclides such as Tc-99m and In-111 are more suitable for scintigraphic imaging. However, it has heretofore been difficult to attach these metallic radionuclides directly to most BAMs because generally there is insufficient affinity between the radionuclide and the BAM. Further, in some cases where such attachment has been possible, the attachment of radionuclide sometimes results in partial or complete loss of the biological activity of the BAM.

For these reasons, it has been proposed by many in the art to radiolabel BAMs with metallic radionuclides by covalent conjugation using a metal chelating agent. For example, Khaw et al., Science 209:295-297 (1980) discloses antibodies to cardiac myosin labeled with In-111 diethylenetriaminepentaacetic acid (DTPA) and use of the labeled antibodies to image for myocardial infarction. Krejcarek et al., Biochem. Biophys. Res. Commun. 77:581-585 (1977) discloses use of DTPA to label proteins such as human serum album (HSA) with metal radionuclides. Pritchard et al., Proc. Soc. Exp. Biol. Med. 151:297-302 (1976) discloses conjugation of antibodies to various agents capable of chelating In-111, such as transferrin, D-penicillamine and deferoxamine. Yokoyama et al., European Patent Application 35,765, published in 1981, discloses deferoxamine as a bifunctional chelator for radiolabeling various BAMs, including proteins (e.g., HSA, urokinase, fibrinogen), antibiotics (e.g., "Bleomycin", "Kanamycin") hormones, saccharides and fatty acids. Haber et al., European Patent Application 38,546 published in 1981, discloses DTPA, ethylenediaminetetraacetic acid (EDTA) and ethylenediamine as bifunctional chelators for radiolabeling proteins, including antibodies, antigens and antibody fragments. Yokoyama et al., U.S. Patent 4,287,362 issued in 1981 discloses 3-carboxy-2-oxopropionaldehyde bis(N-methyl-thiosemicarbazone) (OPBMT) and analogs as bifunctional chelating agents for radiolabeling proteins. Sundberg et al., U.S. Patent 3,994,966, issued in 1976, Meares et al., U.S. Patent 4,043,998 and Leung et al., Int. J. App. Radiation and Isotopes 29:687-692 (1978) disclose bifunctional EDTA analogs such as 1-(p-benzenediazonium)-EDTA and 1-p-aminophenyl-EDTA for protein labeling. Paik et al., J. Radioanal. Chem. 57:553-564 (1980) discloses an azo derivative of DTPA called DTTA-azo-imidate as a bifunctional chelator and its use to label HSA with In-111. Each of the bifunctional chelators heretofore described, however, has generally been designed to coordinate a specific metallic radionuclide. It would, therefore, be desirable to develop a chelator capable of coordinating a variety of metallic cations, and capable of conjugation with BAMs, while retaining the biological activity of the BAMs.

Further, current intravascular radiographic contrast agents are based upon iodinated aromatic compounds. These compounds, however, are often found not to be physiologically tolerable at useful concentrations. Therefore, it would be desirable to develop physiologically compatible alternatives to such iodinated compounds.

Also, in the rapidly developing field of nuclear magnetic resonance (NMR) imaging, useful contrast agents would be valuable, particularly if capable of conjugation with BAMs. Brasch (Radiology, 147:781-788, (1983)) in his review of methods of contrast enhancement for NMR imaging, notes among criteria for the "ideal" contrast enhancer, that the compound should have strong NMR activity at low concentrations, be non-reactive in vivo, and should be non-toxic in diagnostic doses.

Commonly assigned U.S.S.N. 539,733 filed October 6, 1983 in the name of Tolman (hereby incorporated by reference) discloses various processes for preparing conjugates of trace-labeled metallothionein and biologically active molecules (BAMs). Although the processes described therein result in useful trace-labeled conjugates, the yields of those conjugates are somewhat low. There remains, therefore, a need for a process for conjugation of trace-labeled metallothionein to BAMs.

SUMMARY OF THE INVENTION

The instant invention is an improved process for producing tracer-labeled conjugates of metallothionein and BAMs comprising the sequential steps of (i) conjugating the BAM to a heterobifunctional crosslinking agent; and (ii) either (a) conjugating the product of (i) with metallothionein where at least a portion of the metal therein is a tracer metal, or (b) conjugating the product of (i) with metallothionein where all of the metal therein is non-tracer and subsequently exchange labeling at least a portion of the non-tracer metal with tracer metal.

DETAILED DESCRIPTION

Metallothioneins

Metallothioneins are described in Metallothioneins: Proceedings of the First International Meeting on Metallothionein and Other Low Molecular Weight Metal-Binding Proteins, Zurich, July 17-22, 1978, ed. by Kagi and Nordberg, Birkhauser Verlag Base, 1979 (hereinafter Kagi and Nordberg). Pages 46-92 of Kagi and Nordberg are incorporated herein by reference and summarized below. Metallothionein was discovered in 1957; the cadmium and zinc containing protein was isolated from equine kidney. Substantially the same protein was later found in rabbits, humans, monkeys, cattle, sheep, pigs, dogs, hamsters, rats, mice and seals. Equine metallothionein was characterized as having: molecular weight of 6000-7000; high metal content; high cysteine content; generally no aromatic amino acid; and optical features of metal thiolates (mercaptides) and fixed. It was agreed by the plenum of the First International Meeting on Metallothioneins, referred to above, that proteins resembling equine renal metallothionein in several of these features can be designated as "metallothionein" (Kagi and Nordberg, p. 48), and this is the manner in which the term is used in this specification. Of course, metallothionein fragments are also useful in the practice of the subject invention as are functionally similar polypeptides having at least about six amino acid residues.

Generally speaking, metallothioneins are low molecular weight proteins which are produced in vivo and which chelate a wide variety of metal ions with high affinity. The physiological function of metallothioneins is not well-understood, but it is generally accepted that they function in the homeostasis of essential metals and the detoxification of heavy metals. Metallothioneins are ubiquitous to higher vertebrates, invertebrates, and eukaryotic and prokaryotic microorganisms. Exposure of many organisms to metal ions of e.g., Cd, Hg, Zn or Cu induces rapid de novo synthesis of metallothioneins by enhanced production of the mRNA for apoprotein thionein. Therefore, molecules such as cadystin, produced by certain microorganisms in response to Cd injection, are also contemplated for use in the subject invention.

All mammalian thioneins contain 60-61 amino acid residues and can bind 7 gram-atoms of divalent or up to 20 gram-atoms of monovalent metal ion per mole. Thioneins generally contain no aromatic or histidine residues, and 20 of the amino acid residues in mammalian thioneins are cysteines. Based on spectroscopic evidence, metal ligation by thionein is almost exclusively through the sulfhydryl moieties of the cysteines.

Complete amino acid sequences for several metallothioneins have been determined; they are reported on page 60 of Kagi and Nordberg and selected ones are repeated here:

## TABLE 1

### Amino Acid Sequences of Metallothioneins (MT)

```
          1        10       20       30       40       50       60


Human MT-2

Ac-MDPNCSCAAGDSCTCAGSCKCKECKCTSCKKSCCSCCPVGCAKCAGGCICKGASDKCCSCA-OH


Equine MT-1A

Ac-MDPNCSCPTGGSCTCAGSCKCKECRCTSCKKSCCSCCPGGCARCAQGCVCKGASCKCCSCA-OH


Mouse MT-I

Ac-MDPNCSCSTGGSCTCTSSCACKDCKCTSCKKSCCSCCPVGCSKCAQGCVCKGAADKCTCCA-OH


Neurospora MT

H-GDCGCSGASSCNCGSGCSCSNCGSK-OH
```

One-letter symbols:
A  =    Alanine
C  =    Cysteine
D  =    Aspartic acid
E  =    Glutamic acid
G  =    Glycine
I  =    Isoleucine
K  =    Lysine
L  =    Leucine
M  =    Methionine
N  =    Asparagine
P  =    Proline
Q  =    Glutamine
R  =    Arginine
S  =    Serine
T  =    Threonine
V  =    Valine
Other symbols:
Ac  =    Acetyl
H  =     Free amino terminus
OH  =    Free carboxyl terminus

It will be observed that the cysteine residues are distributed along the chain and that there are a number of -C-X-C- residues, where X stands for an amino acid other than cysteine. The table includes a metallothionein from Neurospora crassa with a much lower molecular weight than the mammalian metallothioneins. As reported on p. 55 of Kagi and Nordberg, higher molecular weight metallothioneins (9500-10,000) have been isolated from other microorganisms. All of these metallothioneins are within the scope of the invention, although the mammalian metallothioneins are preferred. For in vivo diagnostic and therapeutic purposes, it is especially preferred to use a metallothionein from the same species as the mammal being treated.

Metal-binding fragments of thionein, for example, as reported in Yoshida et al., Proc. Natl. Acad. Sci., U.S.A 76:486-490, Kondo et al., Tetrahedron Letters, 24:925-928; and Kondo et al., Tetrahedron Letters,

4

25:3869 (1984); and Winge et al., J. Biol. Chem., 257:3471 (1982); all hereby incorporated by reference, can also be used in this invention. The fragments of mouse thionein synthesized by Yoshida et al. have the following amino acid sequences in which the letter symbols have the same meaning as in Table 1 above.

1. $H_2N$-K-C-T-C-C-A-OH

2. $H_2N$-A-C-K-D-C-K-C-T-OH

3. $H_2N$-S-C-T-C-T-S-S-C-A-OH

4. $H_2N$-G-C-S-K-C-A-Q-G-C-V-OH

5. $H_2N$-G-C-V-K-G-A-A-D-K-C-T-C-A-OH

Metal-bound fragments of thioneins (i.e., metallothionein fragments) such as those synthesized by Yoshida et al. are suitable for use in this invention, although complete metallothioneins are presently preferred.

Of course, it will be apparent to those skilled in the art that polypeptides having functional similarities to metallothionein, as well as copolymers of metallothionein/metallothionein fragments and conventional monomers, made using conventional synthetic techniques, are useful in the practice of the subject invention so long as they exhibit the general characteristics of metallothionein as detailed above.

Metallic Trace-Labels

Of the diagnostic and therapeutic radionuclides heretofore known, the following are those useful in the practice of the subject invention (half-life given in d = days, h = hours):

## TABLE II

| Diagnostic Radionuclide | Half life |
| --- | --- |
| Ruthenium-97 | 2.9 d |
| Technetium-99m | 6.0 h |
| Mercury-197 | 2.7 d |
| Gallium-67 | 77.9 h |
| Gallium-68 | 1.1 h |
| Osmium-191 | 15 d |
| Indium-111 | 2.8 d |
| Indium-113m | 1.7 h |
| Lead-203 | 52 h |

| Therapeutic Radionuclide | Half Life |
| --- | --- |
| Palladium-103 | 17.0 d |
| Silver-111 | 7.5 d |
| Antimony-119 | 1.6 d |
| Gold-198 | 2.7 d |
| Copper-67 | 2.6 d |
| Rhenium-188 | 17.0 h |
| Bismuth-212 | 1.0 h |

The diagnostic radionuclides are gamma-emitters and/or positron-emitters, emitting energies between 30KeV and 1MeV and possessing half-lives of between about 1 minute and 8 days. These radionuclides are useful in conjunction with conventional radioscintigraphic imaging techniques based upon, e.g.. planar, single photon or positron tomographic methods. The therapeutic radionuclides emit alpha-, beta-, gamma, conversion electrons or Auger electrons of energies between 100eV and 2MeV, and are capable of killing cells in vivo.

Of course, those skilled in the art will appreciate that when discussing diagnostic and therapeutic uses for these various radionuclides, the dosages utilized will depend upon many variables. When utilizing the radionuclides for imaging purposes, the particular dosage employed need only be high enough to obtain diagnostically useful images, generally in the range of 0.1 to 20 mCi/70 Kg body weight. In contrast, for therapeutic purposes, higher doses can be utilized, generally in the range of 0.1-500 mCi/70 Kg body weight. Of course, the proper dose will ultimately depend on the physical properties of radionuclide such as half-life, type of radiation, and energy of radiation, and on the pharmacokinetics of the radiolabeled agent.

Of the contrast agents heretofore known as being useful for NMR imaging, cobalt, nickel, copper and ruthenium are considered useful in the practice of the subject invention.

Of the contrast agents heretofore known as being useful for radiographic imaging, metallic elements in the periodic table numbered 72 to 83 are considered useful in the practice of the subject invention, and bismuth, lead, mercury, gold, platinum, rhenium and tungsten are preferred.

Target-Seeking Biologically Active Molecules

The term target-seeking biologically active molecules (BAM) as used herein means antibodies (especially monoclonal antibodies), Fab, Fab' and F(ab')$_2$ fragment of antibodies, and other molecules which localize in certain organs, tissues or cells of the mammalian body. Examples of such other molecules are hormones such as insulin, glucagon, prostaglandins, steroidal hormones, and peptides, and other proteins which bind specifically to certain cell types, such as lutenizing hormone which binds to receptors in ovaries. Large molecules such as proteins are generally preferred for radiolabeling through metallothionein conjugation, but conjugation of several small molecules may also be suitable; for example, conjugation to radiolabeled metallothionein of several molecules of quinuclidinyl benzilate, which binds to muscarinic cholinergic receptors of the heart, would provide a radiopharmaceutical for following the viability of the heart cells in vivo. Estrogen and neuropeptides conjugated to radiolabeled metallothioneins could likewise provide breast tumor and brain perfusion agents, respectively.

Conjugation of BAMs to Metallothionein

The improved process of instant invention utilizes crosslinking agents through which BAMs are conjugated to metallothionein. These crosslinking agents are heterobifunctional in nature, i.e. the reactive group used for conjugating the BAM is functionally distinct from the reactive group used for conjugating metallothionein. More specifically, the reactive group of the crosslinking agent to be reacted with metallothionein need be one capable of reaction with the thiol (-SH) group of the metallothionein, while the reactive group of the crosslinking agent to be reacted with the BAM must not be capable of reaction with a thiol group, but must be capable of reaction with another functional group on the BAM. If the BAM is a monoclonal antibody or other glycoprotein, available functional groups will be -NH$_2$, -OH, -CO$_2$H, and NHC-(=NH)NH$_2$. When dealing with smaller drugs or hormones that do not contain such groups, one of these groups may be synthetically incorporated into the small molecule so that conjugation with metallothionein can take place. Methodology will vary widely from one such drug or hormone to another, as is well known to those skilled in the art, a prime consideration being the preservation of biological specificity and affinity. For a general discussion of such synthetic modification, see Means and Feeney, "Chemical Modification of Proteins," Holden-Day, Inc. (1971) hereby incorporated by reference.

Further, the process of this invention requires the BAM to be conjugated to the crosslinking agent before the metallothionein is added to the conjugate. If this sequence is reversed, i.e. if the metallothionein is conjugated to the crosslinking agent prior to introduction of the BAM, undersirable intramolecular and/or intermolecular crosslinking dramatically decreases the yields of BAM - metallothionein conjugates. As seen in the Examples below, the sequential process of the instant invention results in unexpectedly high yields of trace-labeled conjugates of BAMs and metallothionein.

Therefore, the conjugation of BAMs to metallothionein through heterobifunctional crosslinking agents can be represented by the following general formula:

BAM + X-Z-Y    BAM-Z-Y
BAM-Z-Y + MT(-SH)    BAM-Z-MT

where MT(-SH) is metallothionein having -SH groups as the groups to be reacted with Y; X = Y; Z is alkyl, aryl, cycloalkyl, alkenyl, hydroxyalkyl, aminoalkyl, thioalkyl, phosphoalkyl, carboxyalkyl, or keto alkyl of zero (preferably one) to twenty carbon atoms (or appropriately N, S or P atoms) provided that Z does not represent a moiety that will adversely interfere with the desired binding properties of X or Y (Z being zero where X and Y are linked directly to each other); Y is a moiety reactive with thiols including $\alpha$-haloacids, $\alpha$-haloesters, $\alpha$-haloamides, aryl halides and various Michael acceptors such as maleimides, vinyl sulfides and vinyl sulfones; and X is a moiety reactive with non-thiol functional groups on the BAMs such as $-NH_2$, $-OH$, $-CO_2H$ and $-NHC(=NH)NH_2$. Preferred acylating agents for X include activated carboxyl functionalities such as chlorides and anhydrides, imidoesters, thiolesters, and N-hydroxysuccinimide esters. In general, the acylation of amines is preferred over hydroxyls since amides are known to be more stable in vivo than esters. Reductive alkylation of $-NH_2$ groups on the heterobifunctional reagents by aldehyde or ketone groups on the BAM is also useful in this regard.

The preferred moieties for X in forming covalent bonds with $-NHC(=NH)NH_2$ groups include 1,2- and 1,3-dicarbonyl compounds like malondialdehyde, cyclohexane-1,2-dione, and camphorquinone. The reaction of arginine residues with these reagents is very selective and is reversable.

The preferred moiety for X in utilizing carboxy groups of BAMs for crosslinking is the amine group. When activated by a water-soluble carbodiimide such as 1-cyclohexyl-3-(2-morpholinyl-4-ethyl(carbodiimide or 1-ethyl-3-(3-dimethyl-aminopropyl(carbodiimide), the activated carboxyl group of BAMs will react with X = $-NH_2$ of a crosslinking agent to form a amide bond.

Some commercially available crosslinking agents useful in the instant invention include succinimidyl 4-(N-maleimidomethyl) cyclohexane-1-carboxylate (SMCC), succinimidyl 4-(iodoacetyl)aminobenzoate (SIAB), and succinimidyl 4-p-maleimidophenyl)butyrate.

In general, the choice of chain length between X and Y varies depending on the nature of the BAM being attached. With large BAMs, a longer chain may be required to allow optimal covalent bond formation between the crosslinking agent bound to metallothionein and the target functional groups on the large protein. With small BAMs, a very long carbon chain may be required to allow for the BAM attached to metallothionein to interact with its cell surface receptor. The chain length can thus be varied either to optimize the crosslinking process or to maximize the retention of biological activity of the metallothionein-BAM conjugate.

In general, for the conjugation of proteins to metallothionein, mild conditions are required to avoid denaturation of the metallothionein and BAM and loss of biological activity. The pH of the reaction should be in the range of about 3 to 11, preferably 5 to 9, temperature in the range of 0 to 60°C, and concentrations of each BAM and metallothionein in the range of $10^{-2}$ to $10^{-6}$ M, preferably about $10^{-4}$ M. The preferred solvent is generally water although varying amounts of solvents like dimethyl sulfoxide can be added to dissolve non-polar conjugating agents. For conjugating non-proteinaceous BAMs to metallothioneins, somewhat harsher conditions can be tolerated by the metallothionein and reactions can be run in organic solvents like dimethyl sulfoxide.

Trace-Labeling Metallothionein

For purposes of the subject invention, the metallothionein introduced into the reaction scheme above can be "cold" (i.e. not contain any tracer metal) or can be pre-trace labeled (i.e. can have all or a portion of the metal binding sites of metallothionein occupied by tracer metal).

In the situation where trace-labeled metallothionein is to be utilized in the reaction scheme above, there are generally two procedures which can be utilized to produce trace-labeled metallothionein. The first is by direct labeling of thionein. The second is by exchange labeling a metallothionein such as Zn-metallothionein using a desirable metal trace-label.

The first procedure, i.e., the direct labeling of thionein with trace-label, is similar to that reported by M. Vasak and J. Kagi, Proc. Natl. Acad. Sc. USA, 78:6709 (1981) hereby incorporated by reference. Generally, mammalian thionein is dissolved at pH 2 and any resulting insoluble material removed by filtration. To this solution of thionein, the metal trace-label and any desired non-trace-label metal cation are added. The total concentration of trace-label and non-trace-label metal cation should be sufficient to insure that there are available at least seven divalent or twenty monovalent metal cations per thionein, or suitable combinations of divalent and monovalent cations to fill all metal binding sites on the metallothionein. Because of the unique properties of thionein, metallothioneins containing more than one metal cation can be prepared. Therefore, since it is possible that all the metal-binding sites of thionein can be occupied by metal trace-label, trace-labeled metallothioneins of very high concentration can be prepared.

When utilizing a radionuclide as the metal trace-label, the concentration of radionuclide added depends on the specific activity required for the clinical application of interest. For diagnostic applications, the ratio of moles of radionuclide per mole of thionein may be as small as one or less. For therapeutic applications, this ratio may be higher. Non-radioactive metal cations may be added in quantities sufficient to occupy the metal binding sites not occupied by radioactive cations. Following the addition of radionuclides, and optionally non-radioactive metal cation, the resulting solution may be degassed to remove oxygen and neutralized in an inert atmosphere until the pH is greater than 7.0. During this neutralization the thionein folds around the radionuclide and non-radioactive metal cations to form the desired radiolabeled metallothionein. The labeled metallothionein can then be purified by conventional techniques such as dialysis, size exclusion or ion exchange chromatography. The non-radioactive metal content of the radiolabeled metallothionein can be determined by atomic absorption and the radionuclide content assessed by counting radioactive decay. This purified radionuclide-labeled metallothionein can then be coupled directly to a BAM using crosslinking agents described above.

The second labeling procedure involves the exchange of a trace-label metal cation for all or a portion of the non-trace-label metal cations of metallothionein. The success of this exchange reaction requires that the trace-label cation have a higher affinity for the mercaptides of metallothionein than the non-trace-label cation in the preformed metallothionein.

For example, when utilizing radionuclides as the metal trace-label, zinc cations have a lower affinity for the mercaptides of metallothionein than cations of either technetium, mercury, or silver. Therefore, in the presence of cations of technetium-99m, mercury-197, or silver-111, the Zn (II) cations of Zn-metallothionein (MTh) are readily displaced to give $^{99m}$TC,Zn-MTh, $^{197}$Hg,Zn-MTh, or $^{111}$Ag,Zn-MTh, respectively. Thus, since Zn-MTh is easily prepared by the procedure of Vasak et al., supra, exchange labeling of Zn-MTh or a conjugate of Zn-MTh - BAM can be accomplished by mixing Zn-$\overline{\text{MTh}}$ or Zn-MTh-BAM conjugate with a soluble, exchangeable species of radionuclide, e.g., $^{99m}$Tc-glucoheptonate, $^{197}$HgCl$_2$, or $^{111}$Ag(NH$_3$)$_2^+$. Following exchange, the radiolabeled metallothionein can be purified by conventional techniques such as dialysis, size exclusion or ion exchange chromatography. The yield of exchange labeling can be determined by counting the radioactive decay and represents the percentage of total radioactivity which is incorporated into the metallothionein. Cobalt (II), nickel (II), and zinc (II) cations form metallothioneins that can be utilized in an exchange labeling process involving the radionuclides listed in Table 2; however, zinc (II) cations are the preferred non-radioactive cations for this exchange labeling.

Alternatively, "cold" metallothionein can be utilized in the process of the instant invention and exchange-labeled after formation of the BAM- metallothionein conjugate. This exchange labeling is carried out as described above. The use of this exchange labeling procedure is useful especially when utilizing radionuclides with half-lives shorter than 24 hours because this exchange labeling can be carried out at the clinical site. As further discussed below, exchange labeling of a Zn-MTh-BAM conjugate with short-lived radionuclides makes possible the introduction of radionuclides immediately before use and avoids the inevitable loss of radioactivity through decay. Of the radionuclides mentioned above, $^{99m}$Tc is most preferred in this embodiment due to its relatively short half-life, because of its ready availability from Mo-99/Tc-99m generators, and because of the desirable physical characteristics of the radionuclide.

Methods analogous to those described for radiolabeling metallothionein may be used for incorporation of non-radioactive metallic trace-labels. Of those which would be useful for radiographic contrast, i.e., Bi, Rh, Hg, Au, Pt, Re and W, mercury and gold can be incorporated into metallothionein by exchange labeling or by direct conjugation to the apoprotein. Incorporation of the remaining elements is achieved by direct labeling. Of those which would be useful for NMR imaging, i.e., Co, Hi, Cu(II) and Ru, copper (II) and ruthenium is best incorporated into metallothionein by exchange labelling, whereas cobalt and nickel are incorporated by direct labeling.

The trace-labeled conjugates of this invention are used in the same manner as prior art trace-labeled BAMs. They can be lyophilized for storage and shipment then reconstituted in pharmacologically acceptable media, such as normal physiological saline and injected intravenously for diagnostic imaging or therapy, or they can be prepared immediately before use by exchange labeling. They can also be used in in vitro assay and clinical diagnostic methods in the same manner as prior art trace-labeled compounds.

Example 1

Preparation and Evaluation of SIAB-Crosslinked $^{99m}$Tc,Zn-Metallothionein/anti-human Breast Carcinoma B6.2.

A. Preparation of SIAB-Crosslinked $^{99m}$Tc,Zn-MTh-B6.2 Conjugate.

To the monoclonal antibody B6.2 (1.2 mg, 0.7 ml, 8 X 10$^{-6}$ moles) [D. Colcher, et al., Proc. Natl. Acad. Sci., USA, 78, 3199 (1981)] in 0.020 M Hepes pH 7.5 is added a hundred-fold molar excess of sulfosuccinimidyl 4-(iodoacetyl)-aminobenzoate (Sulfo-SIAB, Pierce Chemical Co.) (0.033 ml of a 10 mg/ml distilled water solution). The B6.2 and Sulfo-SIAB are incubated for fifteen minutes at 37$^\circ$ C. The

excess SIAB is removed by Sephadex G-25 fine chromatography (1.0 X 40.0 cm) eluting with 0.05 M TRIS-HCl pH 9.0 The SIAB modified B6.2 (6.0 X $10^{-6}$ mole in 1.6 ml) is recovered from this elution (about 75% protein recovery). To this modified B6.2 is added a twenty-five fold molar excess of Zn-MTh prepared by the method of D. Winge and K. Miklossy, Arch. Biochem. Biophy., 214, 80(1982) in 0.050 M TRIS-HCl pH 9.0 equivalent to 1.5 X $10^{-4}$ mole (0.89 ml of a 1 mg/ml, 1.639 X $10^{-4}$ M stock). After a two-hour incubation at 4°C, the Zn-MTh-SIAB-B6.2 conjugate formation is complete. Unreacted Zn-MTh and other undesired components were removed by Sephadex G-150 chromatography (1.0 X 40.0 cm) eluted with 0.1 M sodium phosphate pH 7.0. To 1 ml of the purified conjugate is added 0.25 ml of 0.4 M monobasic Na phosphate and approximately 0.4 ml of $^{99m}$Tc-GLUCOSCAN™ (120 mCi). Following a one-hour incubation at room temperature, the exchange labeled $^{99m}$Tc,Zn-metallothionein-B6.2 conjugate is purified by HPLC using a BioRad TSK-250 gel filtration column eluted with 0.1 M phosphate buffer at pH 7.0 at 1 ml/min. Following purification 39% of the presented $^{99m}$Tc radioactivity is associated with the SIAB crosslinked conjugate.

B. In Vitro Evaluation of the SIAB-Crosslinked $^{99m}$Tc,Zn-MTh-B6.2 Conjugate.

The effect that conjugation of MT using SIAB has on the ability of the B6.2 to bind the antigen against which it is targeted is assessed by solid phase radioimmunoassay [D. Colcher, M. Zalutsky, W. Kaplan, D. Kufe, F. Austin, F. Schlom, Cancer Research 43:736 (1983)]. This assay employes tumor cell extracts from xenografts grown in athymic mice of the human colorectal adenocarcinoma LS174T [B. H. Tom, et al., In Vitro, 12:180 (1973)]. The LS174T tumor cells possess the antigen to which B6.2 binds, while the A375 cells do not and serve as a control on nonspecific binding of radiolabeled B6.2. Maximal binding of the $^{99m}$Tc-MTh-SIAB-B6.2 in the solid phase assay to the LS174T tumor cell extract is 63% of the $^{99m}$Tc activity added, while binding to the antigen-negative, control A375 tumor extract is 6%. This demonstrates that conjugation of MTh to B6.2 using SIAB results in a conjugate whose specificity for the antigen was generally retained.

Example 2

Preparation of a SMCC-Crosslinked $^{99}$Tc,Zn-MTh-B6.2 Conjugate.

A. Preparation Starting From Zn-MTh (comparative).

The $^{109}$Cd,Zn-MTh is prepared as in Example 1. To the $^{109}$Cd,Zn-MTh (0.366 mg, 0.75 ml, 6 X $10^{-5}$ mM) in 0.02 M Hepes pH 7.5 is added a hundred-fold molar excess of sulfo-succinimidyl 4-(N-maleimidomethyl)cyclohexane 1-carboxylate (Sulfo-SMCC, Pierce Chemical Co.) 2.618 mg). The $^{109}$Cd,Zn-MTh and Sulfo-SMCC are incubated for fifteen minutes at 37°C. The excess Sulfo-SMCC is removed by Sephadex G-25 fine chromatography (1.0 X 40 cm) eluting wtih 0.05 M Na phosphate pH 7.0. SMCC-modified MTh is added to 0.07 molar equivalents of B6.2 (0.5 mg in 0.5 ml, 3.3 X $10^{-6}$ moles) in 0.05 M Na phosphate pH 7.5. After two hours of incubation, conjugate formation is judged complete. The unreacted MTh is removed by Sephadex G-150 chromatography as in Example 1. There is no $^{109}$Cd activity associated with the purified B6.2. The fraction which should be MTh-B6.2 conjugate is exchange labeled as in Example 1. No radioactivity is associated with this material.

B. Preparation Starting From Monoclonal Antibody B6.2 (process of this invention).

To the monoclonal antibody B6.2 (2.5 mg. 1 ml, 1.67 X $10^{-5}$ mole) in 0.020 M Hepes pH 7.5 is added a hundred-fold molar excess of sulfo-succinimidyl 4-(N-maleimidomethyl)cyclohexane 1-carboxylate (Sulfo-SMCC, Pierce Chemical Co.) (0.0729 ml of a 10 mg/ml distilled water stock solution). The B6.2 and Sulfo-SMCC are incubated for fifteen minutes at 37°C. The excess Sulfo-SMCC is removed by Sephadex G-25 fine chromatography (1.0 X 40.0 cm) eluting with 0.050 M TRIS-HCl pH 9.0. The SMCC-modified B6.2 (1.25 X 10-5 mole in 3 ml) is recovered (about 75% protein recovery). To this modified B6.2 is added a twenty-fold molar excess of Zn-MTh prepared as in Example 1 in 0.050 M TRIS-HCl pH 9.0 equivalent to 3.13 X $10^{-4}$ mole (1,85 ml of a 1 mg/ml, 1.639 X $10^{-4}$ M stock). After a two-hour incubation at 4°C, the Zn-MTh-SMCC-B6.2 conjugate formation is complete. Unreacted Zn-MTh and other undesired components are removed by Sephacryl S-300 chromatography (1.0 X 40.0 cm) eluting with 0.020 M Hepes, 0.150 M NaCl pH 7.5. The recovered, purified Ab conjugate is concentrated using a Centricon-30 (Amicon) to 2.1 X $10^{-5}$ mM Ab. To 0.0646 ml of conjugate is added 0.02 ml of 0.4 M monobasic phosphate, 0.025 ml $^{99m}$Tc-GLUCOSCAN™ (5 mCi). Following a four-hour incubation at room temperature, the exchange labeled $^{99m}$Tc,Zn-MTh-B6.2 conjugate is purified by HPLC using a Bio-Rad TSK-250 gel filtration column eluted with 0.1 M phosphate buffer at pH 7.0 at 1 ml/min. Following purification (76%) of the presented $^{99m}$Tc is associated with the SMCC-crosslinked conjugate.

**Claims**

1. A process for producing a trace-labeled conjugate of metallothionein and biologically active molecule (BAM) comprising the sequential steps of:
   (i) conjugating the BAM to a heterobifunctional crosslinking agent; and
   (ii) conjugating the product of (i) with metallothionein where at least a portion of the metal in metallothionein is a tracer metal.

2. A process for producing a conjugate of metallothionein and biologically active molecule (BAM) comprising the sequential steps of:
   (i) conjugating the BAM to a heterobifunctional crosslinking agent;
   (ii) conjugating the product of (i) with metallothionein where all of the metal therein is non-tracer.

3. The process of claim 2 further comprising exchange labeling at least a portion of the non-tracer metal with tracer metal.

4. The process of claim 1 where the BAM is an antibody.

5. The process of claim 1 where the tracer metal is selected from technetium-99m, ruthenium 97, mercury-197, lead-203, palladium-103, silver-111, gold-198, copper-67, rhenium-188, bismuth-212, and elemental cobalt, nickel, copper, ruthenium, bismuth, lead, mercury, gold, platinum, rhenium and tungsten.

6. The process of claims 1, 4 or 5 where the heterobifunctional crosslinking agent has the general formula X-Z-Y where
   Z is alkyl, aryl, cycloalkyl, alkenyl, hydroxyalkyl, aminoalkyl, thioalkyl, phosphoalkyl, carboxyalkyl, or keto alkyl of zero to twenty carbon, nitrogen, sulfur or phosphorus atoms;
   Y is a moiety reactive with thiols; and
   X is a moiety reactive with $-NH_2$, $-OH$, $-CO_2H$, or $-NHC(=NH)NH_2$ groups or aldehydes from oxidized carbohydrate residues.

7. The process of claim 1, 4 or 5 where in the heterobifunctional crosslinking agent is selected from succinimidyl 4-(N-maleimidomethyl)cyclohexane-1-carboxylate; succinimidyl 4-(iodoacetyl) aminobenzoate; and succinimidyl 4-p-maleimidophenyl)butyrate.

8. The process of claim 3 wherein the BAM is an antibody.

9. The process of claim 3 where the tracer metal is selected from technetium-99m, ruthenium 97, mercury-197, lead-203, palladium-103, silver-111, gold-198, copper-67, rhenium-188, bismuth-212, and elemental cobalt, nickel, copper, ruthenium, bismuth, lead, mercury, gold, platinum, rhenium and tungsten.

10. The process of claims 2, 3, 8, or 9 where the heterobifunctional crosslinking agent has the general formula X-Z-Y where
    Z is alkyl, aryl, cycloalkyl, alkenyl, hydroxyalkyl, aminoalkyl, thioalkyl, phosphoalkyl, carboxyalkyl, or keto alkyl of zero to twenty carbon, nitrogen, sulfur or phosphorus atoms;
    Y is a moiety reactive with thiols; and
    X is a moiety reactive with $-NH_2$, $-OH$, $-CO_2H$, or $-NHC(=NH)NH_2$ groups or aldehydes from oxidized carbohydrate residues.

11. The process of claim 2, 3, 8, or 9 where the heterobifunctional crosslinking agent is selected from the process of claim 1, 4 or 5 where in the heterobifunctional crosslinking agent is selected from succinimidyl 4-(N-maleimidomethyl)cyclohexane-1-carboxylate; succinimidyl 4-(iodoacetyl) aminobenzoate; and succinimidyl 4-p-maleimidophenyl)butyrate.

**Revendications**

1. Un procédé de production d'un conjugué marqué isotopiquement de métallothionéine et de molécule

EP 0 196 669 B1

biologiquement active (BAM) comprenant les étapes séquencielles de:
    (i) conjugaison de la BAM avec un agent de réticulation hétérobifonctionnel; et
    (ii) conjugaison du produit de (i) avec une métallothionéine dans laquelle au moins une partie du métal de la métallothionéine est un métal traçant.

2.  Un procédé de production d'un conjugué de métallothionéine et d'une molécule biologiquement active (BAM) comprenant les étapes séquencielles de:
    (i) conjugaison de la BAM avec un agent de réticulation hétérobifonctionnel; et
    (ii) conjugaison du produit de (i) avec une métallothionéine dans laquelle tout le métal contenu dans celle-ci est non-traçant.

3.  Le procédé selon la revendication 2, comprenant de plus le marquage par échange d'au moins' une partie du métal non-traçant avec un métal traçant.

4.  Le procédé selon la revendication 1, dans lequel la BAM est un anticorps.

5.  Le procédé selon la revendication 1, dans lequel le métal traçant est choisi parmi: technetium-99m, ruthenium-97, mercure-196, plomb-203, palladium-103, argent-111, or-198, cuivre-67, rhenium-188, bismuth-212, et les métaux élémentaires cobalt, nickel, cuivre, ruthenium, bismuth, plomb, mercure, or, platine, rhenium et tungstène.

6.  Le procédé selon les revendications 1 ou 4 ou 5, dans lequel l'agent de réticulation hétérobifonctionnel présente la formule générale X-Z-Y dans laquelle:
    Z est un groupe alkyle, aryle, cycloalkyle, alcényle, hydroxyalkyle, aminoalkyle, thioalkyle, phosphoalkyle, carboxyalkyle, ou cétoalkyle de 0 à 20 atomes de carbone, ou un atome d'azote, soufre ou phosphore;
    Y est un groupe réactif avec les thiols; et
    X est un groupe réactif avec les groupes -NH$_2$, -OH, -CO$_2$H ou -NHC(=NH)NH$_2$ ou des aldéhydes issus des résidus carbohydrates oxydés.

7.  Le procédé selon la revendication 1, 4 ou 5, dans lequel l'agent de réticulation hétérobifonctionnel est choisi entre le 4-(N-maléimidométhyl)cyclohexane-1-carboxylate de succinimidyle; le 4-(iodoacétyl)-aminobenzoate de succinimidyle; et le 4-p-(maléimidophényl)butyrate de succinimidyle.

8.  Le procédé selon la revendication 3, dans lequel la BAM est un anticorps.

9.  Le procédé selon la revendication 3, dans lequel le métal traçant est choisi parmi: technetium-99m, ruthenium-97, mercure-197, plomb-203, palladium-103, argent-111, or-198, cuivre-67, rhenium-188, bismuth-212, et les métaux élémentaires cobalt, nickel, cuivre, ruthenium, bismuth, plomb, mercure, or, platine, rhenium et tungstène.

10.  Le procédé selon les revendications 2, 3, 8 ou 9, dans lequel l'agent de réticulation hétérobifonctionnel présente la formule générale X-Z-Y dans laquelle:
    Z est un groupe alkyle, aryle, cycloalkyle, alcényle, hydroxyalkyle, aminoalkyle, thioalkyle, phosphoalkyle, carboxyalkyle, ou cétoalkyle de 0 à 20 atomes de carbone, ou un atome d'azote, soufre ou phosphore ;
    Y est un groupe réactif avec les thiols; et
    X est un groupe réactif avec les groupes -NH$_2$, -OH, -CO$_2$H ou -NHC(=NH)NH$_2$ ou des aldéhydes issus de résidus carbohydrates oxydés.

11.  Le procédé selon la revendication 2, 3, 8 ou 9, dans lequel l'agent de réticulation hétérobifonctionnel est choisi du procédé de la revendication 1, 4 ou 5 où l'agent de réticulation hétérobifonctionnel est choisi parmi le 4-(N-maléimidométhyl)cyclohexane-1-carboxylate de succinimidyle; le 4-(iodoacétyl)-aminobenzoate succinimidyle; et le 4-p-(maléimidophényl)butyrate de succinimidyle.

**Patentansprüche**

1.  Verfahren zur Herstellung eines spurenmarkierten Konjugats von Metallothionein und biologisch aktivem

11

Molekül (BAM), umfassend die aufeinanderfolgenden Schritte
 (i) Konjugieren des BAM an ein heterobifunktionelles Vernetzungsmittel; und
 (ii) Konjugieren des Produkts von (i) mit Metallothionein, wobei wenigstens ein Teil des Metalls im Metallothionein ein Tracer-Metall ist.

2. Verfahren zur Herstellung eines Konjugats von Metallothionein und biologisch aktivem Molekül (BAM), umfassend die aufeinanderfolgenden Schritte
 (i) Konjugieren des BAM an ein heterobifunktionelles Vernetzungsmittel; und
 (ii) Konjugieren des Produkts von (i) mit Metallothionein, wobei alles Metall darin ein Nicht-Tracer ist.

3. Verfahren nach Anspruch 2, des weiteren umfassend das Austauschmarkieren wenigstens eines Teils des Nicht-Tracer-Metalls mit Tracer-Metall.

4. Verfahren nach Anspruch 1, wobei das BAM ein Antikörper ist.

5. Verfahren nach Anspruch 1, wobei das Tracer-Metall ausgewählt ist aus Technetium-99m, Ruthenium-97, Quecksilber-197, Blei-203, Palladium-103, Silber-111, Gold-198, Kupfer-67, Rhenium-188, Bismut-212 und elementarem Cobalt, Nickel, Kupfer, Ruthenium, Bismut, Blei, Quecksilber, Gold, Platin, Rhenium und Wolfram.

6. Verfahren nach Anspruch 1, 4 oder 5, wobei das heterobifunktionelle Vernetzungsmittel die allgemeine Formel X-Z-Y hat, wobei
 Z Alkyl, Aryl, Cycloalkyl, Alkenyl, Hydroxyalkyl, Aminoalkyl, Thioalkyl, Phosphoalkyl, Carboxyalkyl oder Ketoalkyl mit null bis zwanzig Kohlenstoff-, Stickstoff-, Schwefel- oder Phosphor-Atomen ist;
 Y ein gegenüber Thiolen reaktiver Teil ist; und
 X ein gegenüber den Gruppen $-NH_2$, -OH, $-CO_2H$ oder $-NHC(=NH)NH_2$ oder Aldehyden aus oxidierten Kohlenhydrat-Resten reaktiver Teil ist.

7. Verfahren nach Anspruch 1, 4 oder 5, wobei das heterobifunktionelle Vernetzungsmittel ausgewählt ist aus Succinimidyl-4-(N-maleinimidomethyl)cyclohexan-1-carboxylat, Succinimidyl-4-(iodacetyl)-aminobenzoat und Succinimidyl-4-p-maleinimidophenylbutyrat.

8. Verfahren nach Anspruch 3, wobei das BAM ein Antikörper ist.

9. Verfahren nach Anspruch 3, wobei das Tracer-Metall ausgewählt ist aus Technetium-99m, Ruthenium-97, Quecksilber-197, Blei-203, Palladium-103, Silber-111, Gold-198, Kupfer-67, Rhenium-188, Bismut-212 und elementarem Cobalt, Nickel, Kupfer, Ruthenium, Bismut, Blei, Quecksilber, Gold, Platin, Rhenium und Wolfram.

10. Verfahren nach Anspruch 2, 3, 8 oder 9, wobei das heterobifunktionelle Vernetzungsmittel die allgemeine Formel X-Z-Y hat, wobei
 Z Alkyl, Aryl, Cycloalkyl, Alkenyl, Hydroxyalkyl, Aminoalkyl, Thioalkyl, Phosphoalkyl, Carboxyalkyl oder Ketoalkyl mit null bis zwanzig Kohlenstoff-, Stickstoff-, Schwefel- oder Phosphor-Atomen ist;
 Y ein gegenüber Thiolen reaktiver Teil ist; und
 X ein gegenüber den Gruppen $-NH_2$, -OH, $-CO_2H$ oder $-NHC(=NH)NH_2$ oder Aldehyden aus oxidierten Kohlenhydrat-Resten reaktiver Teil ist.

11. Verfahren nach Anspruch 2, 3, 8 oder 9, wobei das heterobifunktionelle Vernetzungsmittel ausgewählt ist aus dem Verfahren nach Anspruch 1, 4 oder 5, worin das heterobifunktionelle Vernetzungsmittel ausgewählt ist aus Succinimidyl-4-(N-maleinimidomethyl)cyclohexan-1-carboxylat, Succinimidyl-4-(iodacetyl)aminobenzoat und Succinimidyl-4-p-maleinimidophenylbutyrat.